# EUROPEAN PATENT SPECIFICATION

(11) **EP 2 790 610 B1**
(45) Date of publication and mention of the grant of the patent: **20.07.2016**
(21) Application number: 12816488.6
(22) Date of filing: 17.12.2012
(51) Int. Cl.: A61F 2/34

(54) **A PROSTHETIC ACETABULAR CUP**
PROTHETISCHE GELENKPFANNE
COTYLE PROTHÉTIQUE

(30) Priority: 15.12.2011 GB 201121529
(43) Date of publication of application: 22.10.2014
(73) Proprietor: Springer, Thorwald, Sheffield, Yorkshire S3 8RN (GB); Grabowski, Marek, 61-508 Poznan (PL)
(72) Inventor: Springer, Thorwald, Sheffield, Yorkshire S3 8RN (GB); Grabowski, Marek, 61-508 Poznan (PL)
(74) Representative: Wilson, Peter
(86) International application number: PCT/GB2012/053159
(87) International publication number: WO 2013/088176

(56) References cited:
- EP-A1- 0 453 694
- EP-A1- 1 181 909
- WO-A1-01/35873
- WO-A1-2004/017870

## Description

The present invention relates to total hip replacement joints and in particular to the acetabular cup used in such a joint.

A hip joint comprises a femoral part having a rounded femoral head which articulates in an acetabular cavity of the pelvis. Both of these parts may require replacing with a prosthetic implant as a result of osteoarthritis, injury or the like. A total hip replacement consists of replacing both the acetabular cavity and the femoral part. The prosthetic implant used in a total hip replacement consists of three parts; the acetabular cup, the femoral component and the prosthetic head.

The acetabular cup is the component which is placed into the acetabular cavity of the pelvis. Cartilage is removed from the acetabular cavity by a suitable reamer to expose cancellous bone and the acetabular cup is typically inserted and secured therein using friction or cement. The cup may be press-fitted into an acetabular cavity having an outer diameter which is slightly less than the cup itself to provide the fricton/interference fit. Alternatively, a cup corresponding in size with the acetabular cavity may be cemented in place. Further alternatively, the exterior surface of a cup may comprise a screw thread which engages with surrounding bone of the acetabular cavity or a cup may be fixed in place by one or more screws which engage the femoral bone when additional stability is required. Depending on the type of cup being used for a particular patient, the cup may be inserted and positioned using a suitable positioning tool, known as an introducer. Where the cup is a press-fit cup, it is then hammered into place to secure the same in the acetabular cavity.

Known acetabular cups typically include one piece plastic cups or metal modular cups. One piece cups are generally polyethylene and are cemented in the acetabular cavity. Cementless metal cups generally consist of two pieces; a shell and a liner. The outer surface of the metal shell comprises a coating to stimulate bone in-growth to help secure the cup in the acetabular cavity. The inside of the shell is designed to receive and support a bearing liner which may typically be polyethylene, ceramic or metal. Polyethylene liners are generally placed in the shell and connected thereto by a rim locking mechanism, whilst ceramic and metal liners are generally attached to the shell with a so-called 'Morse taper'. EP0453694 describes an example known prosthetic acetabular cup.

Total hip replacements are currently the most common orthopaedic operation. However, postoperative complications and problems can include dislocation, loosening, impingement, limited joint movement, joint wear (particularly of the polyethylene liner), infection, osteolysis, metal sensitivity and toxicity, nerve palsy, pain and leg length discrepancy. A known cause of some of these complications is misalignment of the acetabular cup which can occur during insertion and fixation, even when known navigational methods are used. It is known that optimum cup alignment is achieved in less than 50% of hip operations.

Misalignment is caused by the cup shell not being orientated correctly in the acetabular cavity of the pelvis which can be discovered during or after surgery. For example, hammering a press-fit cup into an acetabular cavity is not an accurate means of inserting the cup in the cavity, even if the surgeon was confident alignment had been achieved before insertion. Where misalignment has been discovered during surgery, the cup must be removed, the cavity re-reamed to remove further cartilage and a larger cup inserted, undesirably compromising the integrity of the acetabular cavity and being surgically time consuming. Where such misalignment is discovered after the original operation, revision surgery is undesirably required resulting in the integrity of the bone tissue bordering the cup being compromised, not to mention further inconvenience and increased surgical risks for the patient.

A first aspect of the present invention provides a prosthetic acetabular cup comprising a dome-shaped expandable shell having an annular rim portion and an apex portion and a concave inner cavity extending therebetween for receiving a bearing liner; the shell further comprising an apex aperture centrally disposed in the apex portion relative to a shell axis of rotation and a plurality of radially spaced slots extending from proximal the rim portion to the apex aperture to define a plurality of shell segments expandable outwardly relative to the shell axis about a hinge portion connecting the segments together when an expanding force is applied at the aperture.

Preferably the shell is non-hemispherical when in unexpanded form and is moved towards a hemispherical state when being expanded. Preferably the shell is close to non-hemispherical when in expanded form. The segments are forced to expand outwardly and equally relative to the shell axis about the hinge portion when an expanding force is applied at the aperture so that initial engagement between the shell and preformed bone cavity occurs proximal the hinge portion and is transferred gradually towards the apex portion of the shell during further expansion of the segments. To this effect, the initial pressure against the bone by the expansion of the segments is desirably spread gradually from the vicinity of the hinge portion to the apex portion of the shell. This desirably prevents ejection forces occurring which would otherwise tend to force the cup out of the cavity and enables interference fit forces to be distributed gradually and equally from the opening of the bone cavity towards its base as the shell expands.

The present invention thereby provides for precisely aligned insertion of the cup into a preformed acetabular bone cavity without the need for impact forces otherwise caused by hammering a press-fit cup, for example, which could cause misalignment of the cup and, where the cup needs realigning or replacing, the integrity of the cancellous bone to be compromised. The present invention further provides for equal and balanced transfer of forces from the cup to the bone during installation in the preformed acetabular cavity in an aligned and stable manner. Furthermore, if the shell is not aligned or orientated as desired, the expanding force may be removed from the aperture to allow the shell to elastically return to an unexpanded form. The shell can then be realigned/repositioned and expanded to secure the same in the acetabular cavity without the integrity of the underlying bone being compromised by such realignment/repositioning. This eliminates or at least significantly reduces the problems caused by misalignment occurring post operation which would otherwise undesirably require revision surgery. The present invention further provides a stable and secure platform for bearing liners.

Preferably the expanding force is provided by an expander comprising a conical element complimentarily shaped with the aperture to force the segments apart when inserted therein in an axial direction from the rim portion to the apex portion. Preferably the expander is a frustoconical element comprising an externally disposed screw thread corresponding to a screw thread of the aperture and is adapted to force the segments outwardly when screwed into the aperture.

Suitably the element comprises a throughbore or recess for a suitable tool, such as a screwdriver, Allen™ key or Torx™ drive for example, to engage with and drive the element into or out of the aperture. Preferably the element comprises a throughbore for a tool to engage and/or to view or feel the base of a preformed acetabular cavity to determine the depth of the cup in the cavity during or after installation. Suitably an introducer for initially inserting and aligning the shell in the acetabular cavity may detachably engage with the shell. Preferably such an introducer engages with the aperture or with the throughbore or recess of the element.

Preferably the element comprises an annular shoulder adapted to engage the shell when inserted in the aperture. Preferably the element and shoulder are adapted so that the shoulder completes the base of the shell cavity when the element is inserted in the aperture. Suitably an annular recess may surround the aperture with which the shoulder is complimentarily shaped to engage in. The shoulder and recess interface also suitably acts as a stop to limit the movement of the element in the aperture when a desired final position of the expanded segments has been reached.

Preferably the shoulder provides a locking mechanism between the element and the shell when fully inserted in the aperture. Suitably the annular recess may be bevelled to receive a complimentarily shaped shoulder of the element. A locking mechanism desirably prevents the element from loosening in the aperture post operation.

Suitably the element may be sized according to a desired amount of expansion for a particular acetabular cavity size. For example, an element of larger diameter will cause the shell segments to expand further to engage with a cavity of relatively large diameter compared with an element of smaller diameter which is more suited to a smaller acetabular cavity. The length of the element and/or screw thread characteristics, such as pitch, may also suitably be selected depending on the desired amount and nature of the expansion.

Preferably the slots extend longitudinally over a major part of the shell. Suitably the slots may comprise six primary slots being equally spaced by 60 degrees to each other about the shell axis. Such an arrangement suitably provides six shell segments hingedly connected together by the hinge portion. However, the number of slots and therefore number of segments may suitably be selected depending on the size of the shell and/or flexibility required.

Preferably each segment comprises a secondary slot disposed centrally therein and extending from the hinge portion to a point within limits of the segment. Preferably each secondary slot extends longitudinally relative to the shell axis and terminates at or near a midpoint of its corresponding segment. The secondary slots provide each segment with an additional degree of flexibility.

Preferably each primary and secondary slot comprises a first aperture from which it extends proximal the rim portion. Preferably the first apertures are radially aligned. Suitably the first apertures may define the hinge portion. The first apertures provide each segment with an additional degree of flexibility about the hinge portion. Suitably the first apertures may be circular holes or elongate slots. Preferably the first apertures are lateral slots relative to the shell axis. Suitably the first apertures may have an aspect ratio of about 2:1 in the sense of lateral length:longitudinal height, but of course other aspect ratios may be selected.

Suitably each secondary slot further comprises a secondary aperture at a point along its length. Preferably the secondary aperture of each secondary slot is provided at or near an end distal the first aperture. Preferably the secondary apertures are radially aligned. Such secondary apertures provide the shell segments with additional flexibility about the hinge portion.

Preferably the apex portion of the shell has a wall thickness which is greater than that of the hinge portion. Preferably the wall thickness of the shell increases gradually from the hinge portion to the apex portion. The reduced wall thickness provides the shell with increased flexibility and elasticity around the hinge portion.

Preferably the rim portion of the shell comprises an inwardly extending radial flange. Preferably the flange is adapted to receive and support a bearing liner.

Preferably an outer surface of the shell comprises a bone in-growth stimulating coating to encourage osseointegration.

Also disclosed is a method of implanting an acetabular cup as described above in an acetabular cavity, comprising the steps of:
- preforming the acetabular cavity using a suitable reamer;
- selecting an acetabular cup from a plurality of cups in accordance with the present invention to correspond with the preformed cavity;
- attaching the cup to one end of an introducer;
- inserting and aligning the cup in the cavity;
- applying the expanding force to the aperture to expand the shell segments outwardly to secure the cup in the cavity; and
- detaching the introducer from the cup.

The method may further comprise one or more of the following steps:
- providing an expander to cause the expanding force;
- partially engaging the expander in the aperture before insertion of the cup in the cavity;
- fully engaging the expander in the aperture to force the shell segments outwardly to secure the cup in the cavity;
- disengaging the expander to retract the shell segments;
- repositioning the shell in the cavity before re-engaging the expander in the aperture; and
- providing drive means to engage with the expander and drive the same into or out of the aperture;
- viewing the acetabular cavity through an aperture of the shell to determine the depth of the cup in the cavity during or after insertion; and
- locking the expander in the aperture when the shell is fully expanded.

A further aspect of the present invention provides a prosthetic acetabular cup assembly comprising an acetabular cup as described above, an expander engageable in the central aperture of the shell, and a bearing liner mountable in said shell.

Suitably the expander may be selected from a plurality of differently sized expanders to provide different amounts of shell segment expansion depending on the cavity size and/or bone properties of the patient. Preferably the expander is a frustoconical screw element selected from a plurality of screw elements having different diameters and/or tapers and/or lengths.

The bearing liner may be a suitable liner of preferred size and material according to the shell size and femoral head properties respectively. Suitably the liner may be polyethylene, ceramic or metal. Suitably the liner is compatible with femoral head sizes ranging from preferably 26mm to 36mm in diameter, or other suitable size. Suitably the bearing liner may be selected from a plurality of differently sized liners to correspond with a selected shell size and/or femoral head size. Suitably the liner may be screwed into the shell or attached thereto by suitable means such as a locking mechanism.

Also disclosed is a method of implanting a prosthetic acetabular cup assembly as described above in an acetabular cavity, the method comprising the steps of:
- preforming the acetabular cavity using a suitable reamer;
- attaching the cup to one end of an introducer;
- inserting and aligning the cup in the cavity;
- engaging the expander in the aperture to expand the shell segments outwardly to secure the cup in the cavity;
- detaching the introducer from the cup; and
- mounting the bearing liner in the shell.

Preferably the introducer is adapted to allow a suitable tool, such as screw driver or Allen key, to pass through the same and engage with the expander to drive the same into the aperture of the shell to expand the shell segments.

The method may further comprise one or more of the following steps:
- selecting an acetabular cup from a plurality of cups to correspond with the preformed cavity;
- selecting a bearing liner from a plurality of liners to correspond with the shell and/or a femoral head;
- partially engaging the expander in the aperture before insertion into the cavity;
- driving the expander with a suitable tool into the aperture to expand the shell segments outwardly;
- disengaging the tool from the expander when the segments have been expanded to a desired position;
- viewing the acetabular cavity through an aperture of the shell to determine the depth of the cup in the cavity during or after insertion; and
- locking the expander in the aperture when the shell is fully expanded.

An embodiment of the invention will now be described by way of example only with reference to the accompanying drawings in which:
- Figure 1 shows an underside view of an acetabular cup in accordance with the present invention;
- Figure 2 shows a mid-section through the cup of Figure 1 in retracted and expanded form;
- Figure 3 shows an acetabular cup assembly including the cup of Figures 1 and 2 in expanded form and a bearing liner mounted therein for engagement with a prosthetic femoral head;
- Figure 4 shows side view of an acetabular cup in accordance with a present invention;
- Figure 5 shows acetabular cup in expanded form with a bearing liner removed showing frusto-conical element screwed in and locked;
- Figure 6 shows exploded view of acetabular cup, frusto-conical screw, bearing liner and prosthetic femoral head.

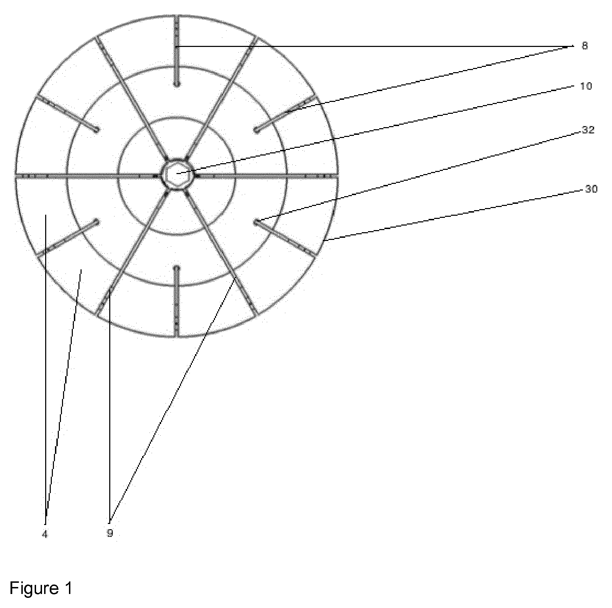

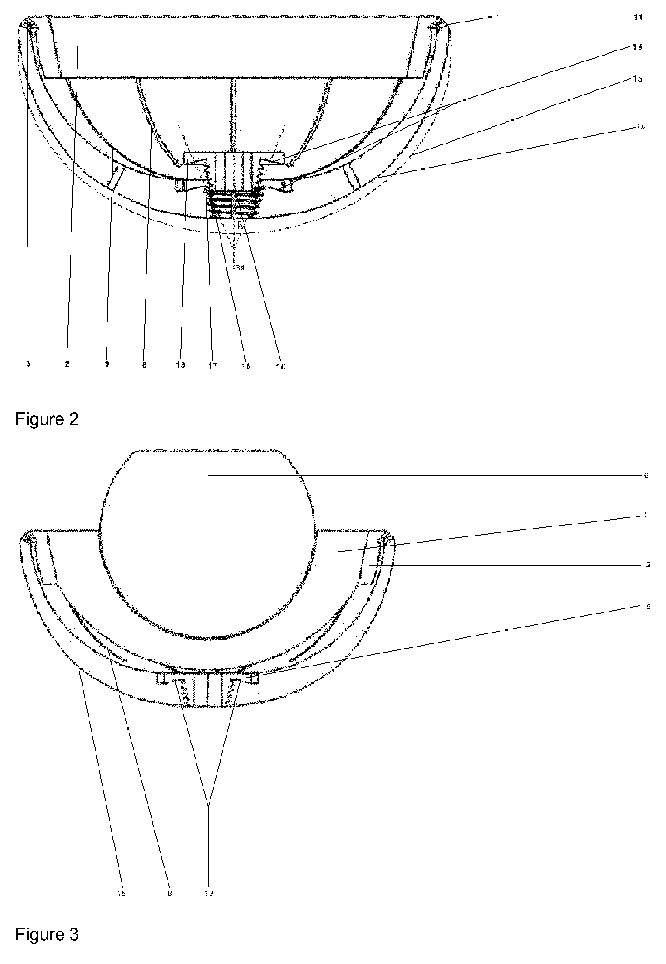

As shown in Figures 1 and 2, an acetabular cup comprises a dome-shaped shell having a rim portion 2 and a central aperture provided in an apex portion of the shell. The shell is formed of bio-compatible metal covered with a bone in-growth supporting coating 7. The outer surface is also roughened to enhance its security in an acetabular cavity and also to provide a key for bone in-growth. The shell is made up of six segments 4 defined by primary slots 9 and secondary slots 8 equally spaced and oriented along meridian planes of the shell. The slots 8 and 9 extend from primary apertures 3 in a sub-equatorial portion of the cup in the vicinity of the rim portion 2 towards the central aperture. The primary slots 9 terminate at the central aperture 21 whereas the secondary slots 8 terminate at secondary apertures 32 provided within the limits of the segments 4. The flexibility of the shell and movement of the segments is provided by slots 8 and 9 and primary apertures 3. The reducing and tapering thickness of the shell wall from the apex portion towards the rim portion provides additional flexibility and elasticity whilst ensuring the shell is sufficiently stiff when in expanded form.

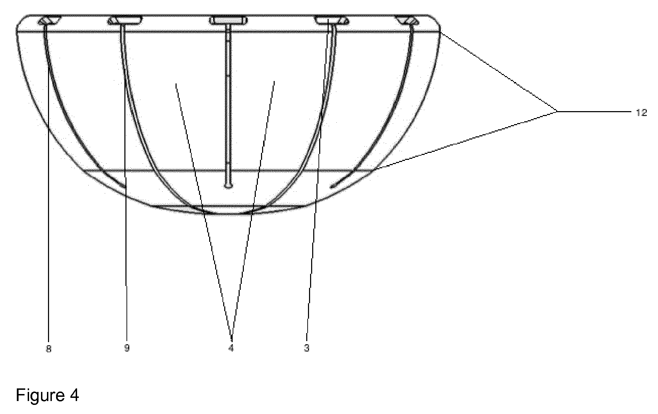

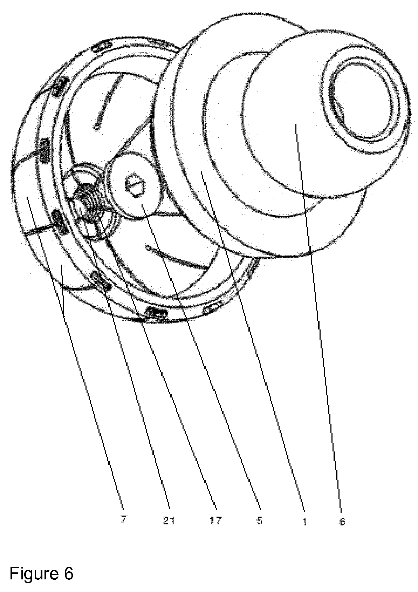

As is further illustrated by the exploded view of figure 6, central aperture 21 comprises a tapered screw thread 17 to receiveably engage with frusto-conical element 5 comprising a corresponding screw thread 18. This arrangement provides an expanding force when screw 5 is driven into aperture 21 to expand the segments outwardly relative to a shell axis 34 of rotation about a hinge portion 11 defined by apertures 3. The conical screw 5 is provided with a central hole 10 correspondingly shaped to receive and engage with a standard Torx™/Allan™ tool (not shown). Outward expansion of the segments from the aperture about a hinge portion proximal the rim portion of the shell advantageously ensures initial engagement between the shell and a preformed bone cavity occurs proximal the hinge portion and is transferred gradually towards the apex portion of the shell during further expansion of the segments at elastic part of the shell 12 as shown in figure 4. To this effect, the initial pressure against the bone by the expansion of the segments is desirably spread gradually from the vicinity of the hinge portion to the apex portion of the shell. This desirably prevents ejection forces occurring which would otherwise tend to force the cup out of the cavity and enables interference fit forces to be distributed gradually and equally from the opening of the bone cavity towards its base as the shell expands. A mechanically stable acetabular cup for receiving a bearing liner is thereby provided.

As shown in Figure 2, the shell is non-hemispherical when in retracted form, as shown by the solid lines 14, whilst being substantially hemispherical when the shell segments have been fully expanded, as shown by the dashed lines 15. Expansion of the segments is limited by a locking mechanism provided by bevelled shoulder 19 on the element 5 which correspondingly engages in bevelled recess 19 of the shell apex portion. Such a locking mechanism desirably ensures the element 5 does not loosen and disengage with the central aperture post operation causing shell segments to retract. The range of acetabular cup expansion and thus interference fit fixation grade can be modified depending on the specific application of the cup by selectively varying angle β of frusto-conical screw 5. The length and/or screw thread dimensions may also be selected depending on the amount and rate of expansion required for a particular cavity size and/or bone property. An element 5 may be selected from a plurality of differently sized elements to engage with a shell selected from a plurality of differently sized shells for engaging with differently sized cavities and/or receiving differently sized bearing liners.

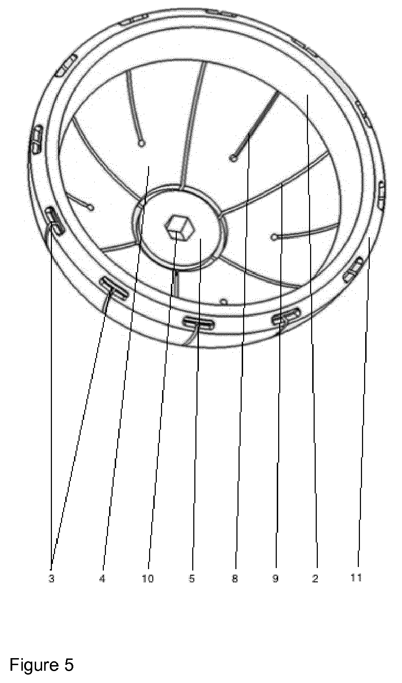

Figure 3 shows an acetabular cup in a fully expanded and locked position including an acetabular cup liner 1 suitably mounted on the rim portion 2 with an articulating prosthetic femoral head 6 engaged therein. As described above, the final, fully expanded position of the shell is defined by the frusto-conical screw 5 being fully screwed into the central aperture 21 and being secured by full engagement of the locking collar 13 into recess 19 of the shell. The above acetabular cup configuration is also shown in Figure 5 with bearing liner removed showing frusto-conical screw fully engaged and locked.

The present invention provides a simple, correctable and mechanically stable means of inserting and securing a prosthetic acetabular cup in an acetabular bone cavity in a non-intrusive and aligned manner eliminating the need for high impact insertion forces and post operation corrective surgery. The present invention also provides a universal cup for cavities of different sizes/bone properties which further provides a stable and secure platform for an acetabular cup bearing liner to be mounted in.

## Claims

1. A prosthetic acetabular cup comprising a dome-shaped expandable shell having an annular rim portion (2) and an apex portion and a concave inner cavity extending therebetween for receiving a bearing liner; the shell further comprising an apex aperture (10, 21); **characterised in that** the aperture is centrally disposed in the apex portion relative to a shell axis of rotation and **in that** a plurality of radially spaced slots (9) extend from proximal the rim portion to the apex aperture to define a plurality of shell segments (4) expandable outwardly relative to the shell axis about a hinge portion (11) connecting the segments (4) together when an expanding force is applied at the aperture.

2. A prosthetic acetabular cup according to claim 1, wherein the shell is non-hemispherical when in unexpanded form and relatively close to hemispherical when in expanded form.

3. A prosthetic acetabular cup according to claim 1 or 2, wherein the expanding force is provided by an expander comprising a conical element complimentarily shaped with the aperture (10, 21) to force the segments (4) apart when inserted therein in an axial direction from the rim portion to the apex portion.

4. A prosthetic acetabular cup according to claim 3, wherein the expander is a frustoconical element comprising an externally disposed screw thread corresponding to a screw thread (17) of the aperture and is adapted to force the segments outwardly when screwed into the aperture.

5. A prosthetic acetabular cup according to claim 4, wherein the element comprises a throughbore or recess for a suitable tool to engage with and drive the element into or out of the aperture.

6. A prosthetic acetabular cup according to claim 5, wherein the throughbore provides means to view or feel the base of a preformed bone cavity to determine the depth of the cup in the cavity during or after installation.

7. A prosthetic acetabular cup according to any preceding claim, wherein the shell is adapted to detachably engage with an introducer for initially inserting and aligning the shell in the acetabular cavity.

8. A prosthetic acetabular cup according to any preceding claim, wherein the element comprises an annular shoulder (19) adapted to engage the shell when inserted in the aperture.

9. A prosthetic acetabular cup according to any preceding claim, wherein each segment (4) comprises a secondary slot (8) disposed centrally therein and extending from the hinge portion (11) to a point within limits of the segment.

10. A prosthetic acetabular cup according to any preceding claim, wherein the apex portion of the shell has a wall thickness which is greater than that of the hinge portion (11).

11. A prosthetic acetabular cup according to any preceding claim, wherein the rim portion (2) of the shell comprises an inwardly extending radial flange for receiving and supporting a bearing liner.

12. A prosthetic acetabular cup according to any preceding claim, wherein an outer surface of the shell comprises a bone in-growth stimulating coating (7).

13. A prosthetic acetabular cup assembly comprising an acetabular cup according to any one or more of claims 1 to 12, an expander engageable in the central aperture of the shell, and a bearing liner mountable in said shell.

14. A prosthetic acetabular cup assembly according to claim 13, wherein the expander is a frustoconical screw element selected from a plurality of screw elements having different diameters and/or tapers and/or lengths and wherein the element is engageable with the central aperture of the shell comprising a corresponding screw thread.

15. A prosthetic acetabular cup assembly according to claim 13 or 14, wherein the liner is polyethylene, ceramic or metal.

## Patentansprüche

1. Eine prothetische Acetabulumpfanne, beinhaltend eine kuppelförmige expandierbare Schale mit einem ringförmigen Randabschnitt (2) und einem Apexabschnitt und einer konkaven inneren Aushöhlung, die sich zwischen diesen erstreckt, zum Aufnehmen eines Auflageeinsatzes; wobei die Schale ferner eine Apexöffnung (10, 21) beinhaltet; **dadurch gekennzeichnet, dass** die Öffnung in dem Apexabschnitt relativ zu einer Schalendrehachse mittig angeordnet ist und dass sich eine Vielzahl von radial beabstandeten Schlitzen (9) von nahe dem Randabschnitt zu der Apexöffnung erstreckt, um eine Vielzahl von Schalensegmenten (4) zu definieren, die, wenn eine Expandierkraft auf die Öffnung angewendet wird, relativ zu der Schalenachse um einen Gelenkabschnitt (11), der die Segmente (4) miteinander verbindet, nach außen expandierbar sind.

2. Prothetische Acetabulumpfanne gemäß Anspruch 1, wobei die Schale in der nicht expandierten Form nicht hemisphärisch ist und in der expandierten Form nahezu hemisphärisch ist.

3. Prothetische Acetabulumpfanne gemäß Anspruch 1 oder 2, wobei die Expandierkraft von einer Expandiervorrichtung bereitgestellt wird, beinhaltend ein konisches Element, das komplementär zu der Öffnung (10, 21) geformt ist, um beim Einführen in diese in einer axialen Richtung von dem Randabschnitt zu dem Apexabschnitt die Segmente (4) auseinander zu drängen.

4. Prothetische Acetabulumpfanne gemäß Anspruch 3, wobei die Expandiervorrichtung ein kegelstumpfförmiges Element ist, beinhaltend ein extern angeordnetes Schraubengewinde, das einem Schraubengewinde (17) der Öffnung entspricht, und angepasst ist, um beim Schrauben in die Öffnung die Segmente nach außen zu drängen.

5. Prothetische Acetabulumpfanne gemäß Anspruch 4, wobei das Element eine Durchgangsbohrung oder eine Aussparung für ein geeignetes Werkzeug zum Eingreifen in das Element und Treiben von diesem in die Öffnung oder aus dieser heraus beinhaltet.

6. Prothetische Acetabulumpfanne gemäß Anspruch 5, wobei die Durchgangsbohrung ein Mittel zum Betrachten oder Fühlen der Basis einer vorgebildeten Knochenaushöhlung beinhaltet, um die Tiefe der Pfanne in der Aushöhlung während oder nach der Einrichtung zu bestimmen.

7. Prothetische Acetabulumpfanne gemäß einem der vorhergehenden Ansprüche, wobei die Schale zum lösbaren Eingreifen in einen Inserter zum anfänglichen Einführen und Ausrichten der Schale in der Acetabulumaushöhlung angepasst ist.

8. Prothetische Acetabulumpfanne gemäß einem der vorhergehenden Ansprüche, wobei das Element eine ringförmige Schulter (19) beinhaltet, die angepasst ist, um beim Einführen in die Öffnung in die Schale einzugreifen.

9. Prothetische Acetabulumpfanne gemäß einem der vorhergehenden Ansprüche, wobei jedes Segment (4) einen sekundären Schlitz (8) beinhaltet, der mittig in diesem angeordnet ist und sich von dem Gelenkabschnitt (11) zu einem Punkt innerhalb Grenzen des Segments erstreckt.

10. Prothetische Acetabulumpfanne gemäß einem der vorhergehenden Ansprüche, wobei der Apexabschnitt der Schale eine Wanddicke aufweist, die größer als die des Gelenkabschnitts (11) ist.

11. Prothetische Acetabulumpfanne gemäß einem der vorhergehenden Ansprüche, wobei der Randabschnitt (2) der Schale einen sich nach innen erstreckenden radialen Flansch zum Aufnehmen und Stützen eines Auflageeinsatzes beinhaltet.

12. Prothetische Acetabulumpfanne gemäß einem der vorhergehenden Ansprüche, wobei eine äußere Oberfläche der Schale eine Knocheneinwuchsstimulierungsbeschichtung (7) beinhaltet.

13. Eine prothetische Acetabulumpfannenanordnung, beinhaltend eine Acetabulumpfanne gemäß einem oder mehreren der Ansprüche 1 bis 12, eine in die mittige Öffnung der Schale eingreifbare Expandiervorrichtung und einen in der Schale montierbaren Auflageeinsatz.

14. Prothetische Acetabulumpfannenanordnung gemäß Anspruch 13, wobei die Expandiervorrichtung ein kegelstumpfförmiges Schraubenelement ist, ausgewählt aus einer Vielzahl von Schraubenelementen mit unterschiedlichen Durchmessern und/oder Verjüngungen und/oder Längen, und wobei das Element in die mittige Öffnung der Schale, beinhaltend ein entsprechendes Schraubengewinde, eingreifen kann.

15. Prothetische Acetabulumpfannenanordnung gemäß Anspruch 13 oder 14, wobei der Einsatz Polyethylen, Keramik oder Metall ist.

## Revendications

1. Un cotyle prothétique comprenant une coque dilatable en forme de dôme ayant une portion de bord annulaire (2), une portion de sommet et une cavité interne concave se prolongeant entre les deux et destinée à recevoir une garniture d'appui ; la coque comprenant de plus une ouverture au sommet (10, 21) ; **caractérisé en ce que** l'ouverture est disposée de façon centrale dans la portion de sommet par rapport à un axe de rotation de la coque et **en ce qu'**une pluralité de fentes espacées radialement (9) se prolongent de la partie proximale de la portion de bord jusqu'à l'ouverture au sommet définissant une pluralité de segments de coque (4) pouvant s'étendre vers l'extérieur par rapport à l'axe de la coque autour d'une portion formant charnière (11) connectant les segments (4) ensemble quand une force de dilatation est appliquée au niveau de l'ouverture.

2. Un cotyle prothétique selon la revendication 1, dans lequel la coque n'est pas hémisphérique lorsqu'elle est sous forme non dilatée et est relativement proche d'une forme hémisphérique lorsqu'elle est sous forme dilatée.

3. Un cotyle prothétique selon la revendication 1 ou la revendication 2, dans lequel la force de dilatation est exercée par un élargisseur comprenant un élément conique de forme complémentaire à celle de l'ouverture (10, 21) pour écarter de force les segments (4) quand il est inséré à l'intérieur dans une direction axiale à partir de la portion de bord jusqu'à la portion de sommet.

4. Un cotyle prothétique selon la revendication 3, dans lequel l'élargisseur est un élément tronconique comprenant un filetage disposé sur l'extérieur correspondant à un filetage (17) de l'ouverture et est adapté pour forcer les segments vers l'extérieur lorsqu'il est vissé dans l'ouverture.

5. Un cotyle prothétique selon la revendication 4, dans lequel l'élément comprend un trou de passage ou un logement pour qu'un outil adapté se mette en prise avec l'élément et le fasse rentrer dans ou sortir de l'ouverture.

6. Un cotyle prothétique selon la revendication 5, dans lequel le trou de passage fournit des moyens pour voir ou pour toucher la base d'une cavité osseuse préformée afin de déterminer la profondeur de la cupule dans la cavité durant ou après installation.

7. Un cotyle prothétique selon n'importe quelle revendication précédente, dans lequel la coque est adaptée pour se mettre en prise de manière amovible avec un élément d'introduction pour l'insertion initiale et l'alignement de la coque dans la cavité acétabulaire.

8. Un cotyle prothétique selon n'importe quelle revendication précédente, dans lequel l'élément comprend un épaulement annulaire (19) adapté pour se mettre en prise avec la coque quand il est inséré dans l'ouverture.

9. Un cotyle prothétique selon n'importe quelle revendication précédente, dans lequel chaque segment (4) comprend une fente secondaire (8) disposée de manière centrale à l'intérieur du segment et qui se prolonge de la portion formant charnière (11) jusqu'à un point au sein du segment.

10. Un cotyle prothétique selon n'importe quelle revendication précédente, dans lequel la portion de sommet de la coque a une épaisseur de paroi plus importante que la portion formant charnière (11).

11. Un cotyle prothétique selon n'importe quelle revendication précédente, dans lequel la portion de bord (2) de la coque comprend une bride radiale se prolongeant vers l'intérieur destinée à recevoir et à soutenir une garniture d'appui.

12. Un cotyle prothétique selon n'importe quelle revendication précédente, dans lequel une surface externe de la coque comprend un revêtement stimulant la croissance osseuse (7).

13. Un ensemble formant cotyle prothétique comprenant un cotyle selon une quelconque ou plusieurs des revendications 1 à 12, un élargisseur pouvant être mis en prise dans l'ouverture centrale de la coque, et une garniture d'appui peut être installée dans ladite coque.

14. Un ensemble formant cotyle prothétique selon la revendication 13, dans lequel l'élargisseur est un élément de vissage tronconique sélectionné dans une pluralité d'éléments de vissage ayant différents diamètres et/ou différentes conicités et/ou longueurs et dans lequel l'élément peut être mis en prise avec l'ouverture centrale de la coque comprenant un filetage correspondant.

15. Un ensemble formant cotyle prothétique selon la revendication 13 ou la revendication 14, dans lequel la garniture est du polyéthylène, de la céramique ou du métal.
